# EUROPEAN PATENT APPLICATION

(11) **EP 4 449 888 A1**
(43) Date of publication of application: **23.10.2024**
(21) Application number: 22907906.6
(22) Date of filing: 13.12.2022
(51) Int. Cl.: A23L 27/50, A23L 11/50

(54) **METHOD FOR PREPARING VEGETABLE FERMENTATION PRODUCT PROVIDING DEEP FLAVORS AND HAVING ENHANCED GAMMA-GLUTAMYL PEPTIDE**

(30) Priority: 14.12.2021 KR 20210178943
(71) Applicant: Sempio Foods Company, Seoul 04557 (KR)
(72) Inventor: HAN, Song Yi, Cheongju-si, Chungcheongbuk-do 28160 (KR); HURH, Byung-serk, Sejong 30101 (KR); CHOI, Yong Ho, Hwaseong-si, Gyeonggi-do 18466 (KR); LEE, Yun Jeong, Cheongju-si, Chungcheongbuk-do 28118 (KR); LEE, Jae Jung, Sejong 30101 (KR); LEE, Song Jae, Cheongju-si, Chungcheongbuk-do 28412 (KR)
(74) Representative: Schön, Christoph
(86) International application number: PCT/KR2022/020268
(87) International publication number: WO 2023/113439

(57) **Abstract**

Disclosed in the present specification are a vegetable fermentation product comprising a high concentration of gamma-glutamyl peptide, and a method for preparing the vegetable fermentation product. The vegetable fermentation product of the present disclosure according to one aspect comprises a high concentration of gamma-glutamyl peptide to provide deep and rich flavors, and may be used in sauce such as soy sauce to add deep flavors to foods. Further, by using the method for preparing the vegetable fermentation product according to one aspect of the present disclosure, a vegetable fermentation product comprising a high concentration of gamma-glutamyl peptide can be prepared in a stable manner.

## Description

### [Technical Field]

The present disclosure relates to a plant-based fermented product comprising a high concentration of gamma-glutamyl peptide and a method for preparing the plant-based fermented product.

### [National R&D project that sponsored this invention]

[Unique Task Identification Number] 1545024061
[Task Identification Number] 120024022HD
[Name of Ministry] Ministry of Agriculture, Food and Rural Affairs
[Name of Project Management (Professional) Organization] Korea Institute of Agriculture, Food and Rural Technology Planning and Evaluation
[Research Project Name] Development of customized innovative food and natural safe material technology (R&D)
[Research Task Name] Development of plant-based fermented flavor modules 1 and 2
[Contribution Rate] 3/5
[Name of Project-Performing Organization] Sempio Foods Co., Ltd.
[Research Period] 2021.01.01 ~ 2021.12.31

### [National R&D project that sponsored this invention]

[Unique Task Identification Number] 1711129879
[Task Identification Number] 2017M3C1B5019298
[Ministry Name] Ministry of Science and ICT
[Name of Project Management (Professional) Organization] National Research Foundation of Korea
[Research Project Name] STEAM Research (R&D)
[Research Task Name] Development of fermented soybean products focused on Use value
[Contribution Rate] 1/5
Name of Project-Performing Organization] Sempio Foods Co., Ltd.
[Research Period] 2021.01.01 ~ 2021.12.31

### [National R&D project that sponsored this invention]

[Unique Task Identification Number] 1545026165
[Task Identification Number] 322022051SB010
[Name of Ministry] Ministry of Agriculture, Food and Rural Affairs
[Name of Project Management (Professional) Organization] Korea Institute of Agriculture, Food and Rural Technology Planning and Evaluation
[Research Project Name] Development of high value-added food technology
[Research Task Name] Development of global natural seasoning materials using Jang fermentation technology
[Contribution Rate] 1/5
[Host Organization] Fermentation R&D Center, Sempio Foods Co., Ltd.
[Research Period] 2022.04.01 ~ 2022.12.31

### [Background Art]

In the Joseon Dynasty's agricultural book, Jeungbosalrimgyeongje ( 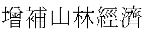 ) (1766), there is an expression "Sauce ( 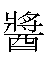 ) is Jang ( 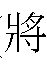 )" meaning that "the sauce ( 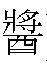 ) is the best of all tastes." <Gyuhapchongseo ( 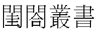 )〉 (1815), a cookbook from the Joseon Dynasty, teaches a wise saying of "Eat rice like spring, eat soup like summer, eat sauce ( 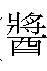 ) like autumn, and drink alcohol like winter," which shows how important the taste of soy sauce played a role in the dietary life of our people.

The taste of soy sauce can be said to be a Gippeunmat consisting of various flavor components created during a fermentation process. The Gippeunmat is a well-aged savory flavor, and has a deep and rich taste obtained by cooking a lot of food materials for a long time. The Gippeunmat makes it possible to enjoy a natural taste of the food materials by enhancing a positive taste and aroma of the food materials and reducing a negative taste and aroma (bitterness, fishy aroma, etc.). This is a clean and light taste that is different from the stimulating and greasy artificial flavor felt when using an artificial flavoring material.

In Japan, there is an expression called Koku ( 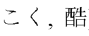 ) similar to the Gippeunmat, which is considered an important factor in improving palatability of a food product. The Koku is a unique sensation that induces various stimuli of tastes, aromas, and textures, and is expressed as complexity. When this sensation spreads throughout the mouth while maintaining its unique flavor (density, richness, mouthfulness) and a lingering taste (continuity) remains even after swallowing, it is expressed as Koku.

In Japan, a lot of research has been conducted on substances having Koku properties, and among these, gamma-glutamyl peptide such as glutathione and γ-EVG is known to be effective even at its low concentration. The gamma-glutamyl peptide is known to improve sweetness, saltiness, and umami as well as the Koku properties by acting as an agonist on CaSR (calcium-sensing receptor) present in taste cells.

The gamma-glutamyl peptide is widely present in nature, and has been found to be present in food products, especially fermented food products such as soy sauce, cheese, and salted fish. Microorganisms such as *Penicillium roquefortii, Bacillus* spp. and lactic acid bacteria used in the fermented food products produce proteolytic enzymes and gamma-glutamyltransferases which are necessary for bioconversion of the gamma-glutamyl peptide. During the fermentation process, proteins as the raw material are hydrolyzed into amino acids and peptides by the proteolytic enzymes, and are bio-converted into the gamma-glutamyl peptide by being used as a donor and acceptor substrate by action of the gamma-glutamyltransferases.

Synthesis of conventional gamma-glutamyl peptide is carried out under an environment where only single amino acid and peptide-typed substrates and purely separated/purified enzymes produced by genetically recombined microorganisms exist, so that there are few factors that interfere with the synthesis to allow the synthesis to carry out at high concentration in a short period of time and a content of the gamma-glutamyl peptide is very high because the synthesis is accomplished in a short time under a condition having maximum synthesis yield. Meanwhile, a plant-based protein material contains complex components such as carbohydrates in addition to the proteins, which act as an obstacle to bioconversion of the gamma-glutamyl peptide, thereby making it difficult to produce a fermented food product with gamma-glutamyl peptide of a high content.

Further, the plant-based protein materials contain a large amount of amino acids such as glutamine that can be used as a substrate for the gamma-glutamyl peptide, but there is a problem that they cannot be directly used as the substrate because they exist in a form of the protein. In order for the plant-based proteins to be used as the substrate, a long-term fermentation process is required to hydrolyze them into free amino acids and low molecular peptides. The gamma-glutamyl peptide already produced during this process may be bio-converted into other substances by action of the gamma-glutamyltransferase, making it difficult to produce a fermented food products with the gamma-glutamyl peptide of a high content.

There have been no known food products that have significantly increased content of the gamma-glutamyl peptide through bioconversion of the plant-based protein materials using the microbial fermentation technology. The gamma-glutamyl peptide analyzed in conventional fermented food products includes γ-EE (18.4-25.2 mg/L), γ-EI (11.3-13.4 mg/L), γ-EL (11.6-14.0 mg/L), γ- EF (30.3-31.5 mg/L), and γ-EVG (0.15-0.61 mg/dl), which have very small contents.

Leucine aminopeptidase is an enzyme that hydrolyzes amino acids located at aN-end of the peptide. Since the leucine aminopeptidase has a wide substrate specificity, unlike most enzymes that have the substrate specificity of a narrow range, it is possible to cleave the bonds of various amino acids located at the N-end of the peptide. Therefore, during fermentation under an environment where the leucine aminopeptidase and the gamma-glutamyltransferase have a high activity, the plant-based proteins can be rapidly hydrolyzed into free amino acid and low molecular peptide forms, which are substrates for the gamma-glutamyltransferase, and bio-converted into gamma-glutamyl peptide form to produce a high concentration of the gamma-glutamyl peptide.

Accordingly, while seeking a way to bio-convert plant-based proteins into gamma-glutamyl peptides, the present inventors have found that a plant-based fermented product comprising a high concentration of gamma-glutamyl peptide can be produced by fermenting a plant-based protein material using *Aspergillus oryzae* strain with leucine aminopeptidase of an high activity and *Bacillus amyloliquefaciens* strain with gamma-glutamyltransferase of a high activity; and have completed the present disclosure.

### [Disclosure]

### [Technical Problem]

In one aspect, the present disclosure is to provide a plant-based fermented product comprising a high concentration of gamma-glutamyl peptide.

In another aspect, the present disclosure is to provide a method for preparing a plant-based fermented product comprising a high concentration of gamma-glutamyl peptide using of *Aspergillus oryzae* strain and *Bacillus amyloliquefaciens* strain.

In still another aspect, the present disclosure is to provide a food composition comprising the plant-based fermented product.

### [Technical Solution]

In order to achieve the above problem, in one aspect, the present disclosure provides a plant-based fermented product comprising gamma-glutamyl peptide (γ-glutamyl peptide) at a concentration of 700 ppm or more.

In an exemplary embodiment, the gamma-glutamyl peptide may include one or more of gamma-glutamyl dipeptide of γ-Glu-X and gamma-glutamyl tripeptide of γ-Glu-X-Y.

In an exemplary embodiment, X and Y as described above may be the same or different amino acids.

In an exemplary embodiment, the amino acids may be selected from the group consisting of aspartic acid (Asp), threonine (Thr), serine (Ser), asparagine (Asn), glutamic acid (Glu), glutamine (Gln), cysteine (Cys), proline (Pro), glycine (Gly), alanine (Ala), valine (Val), methionine (Met), isoleucine (Ile), leucine (Leu), tyrosine (Tyr), phenylalanine (Phe), histidine (His), tryptophan (Trp), lysine (Lys), and arginine (Arg).

In an exemplary embodiment, the fermented product may be any one or more fermented products selected from the group consisting of soybeans, peas, chickpeas, lentils, Fava beans, mung beans, rice, and purified proteins thereof.

In an exemplary embodiment, the fermented product may be a fermented product of *Aspergillus oryzae* strain, and the *Aspergillus oryzae* strain may be an *Aspergillus oryzae* SMF 119 strain (deposit number KCTC12947BP).

In an exemplary embodiment, the fermented product may be a fermented product of *Bacillus amyloliquefaciens* strain, and the *Bacillus amyloliquefaciens* strain may be a *Bacillus amyloliquefaciens* strain SMB469 (deposit number KCTC12822BP).

In one aspect, the present disclosure provides a food composition comprising the plant-based fermented product.

In one aspect, the present disclosure provides a flavoring material comprising the plant-based fermented product.

In an exemplary embodiment, the plant-based fermented product may be contained in an amount of 0.001 to 99.99 % by weight based on the total weight of the flavoring material.

In one aspect, the present disclosure provides a method for preparing a plant-based fermented product, comprising the steps of: inoculating and culturing *Aspergillus oryzae* strain with a plant-based material; and inoculating *Bacillus amyloliquefaciens* strain to the cultured plant-based material and fermenting it.

In an exemplary embodiment, the *Aspergillus oryzae* strain may be *Aspergillus oryzae* SMF119 strain, and the *Aspergillus oryzae* SMF119 strain may have a deposit number of KCTC12947BP.

In an exemplary embodiment, the step of inoculating and culturing the *Aspergillus oryzae* strain may be performed at 20 to 40°C for 40 to 72 hours.

In an exemplary embodiment, the *Bacillus amyloliquefaciens* strain may be a *Bacillus amyloliquefaciens* SMB469 strain, and the *Bacillus amyloliquefaciens* SMB469 strain may have a deposit number of KCTC12822BP.

In an exemplary embodiment, the step of inoculating and fermenting the *Bacillus amyloliquefaciens* strain may be performed at 25 to 55°C for 5 to 200 days.

In an exemplary embodiment, the fermenting step may be characterized by adding brine to the cultured plant-based material.

In an exemplary embodiment, the method for preparing the plant-based fermented product may further comprise heat treating the plant-based material before the culturing step.

### [Advantage Effects]

In one aspect, a plant-based fermented product according to the present disclosure can comprise a high concentration of gamma-glutamyl peptide to produce deep and rich flavors. The plant-based fermented product can be used as a source such as soy sauce to impart Gippeunmat to a food product.

In one aspect, a plant-based fermented product according to the present disclosure can reduce sodium intake by imparting Gippeunmat to the food product with sodium of a low content to raise palatability.

In one aspect, according to a method for preparing the plant-based fermented product according to the present disclosure, the plant-based fermented product comprising a high concentration of gamma-glutamyl peptide can be prepared by effectively hydrolyzing proteins from a plant-based material into free amino acids and dipeptides using *Aspergillus oryzae* strain with leucine aminopeptidase of a high activity and fermenting the proteins using *Bacillus amyloliquefaciens* strain with gamma-glutamyltransferase of a high activity.

### [Description of the Drawings]

FIG. 1 is a graph showing gamma-glutamyl peptide contents between a plant-based fermented product according to an example of the present disclosure and that of Comparative Examples.

FIG. 2 is a graph showing the results of sensory evaluation of soup prepared by a plant-based fermented product according to an example of the present disclosure.

### [Mode for Invention]

Hereinafter, the present disclosure will be described in detail.

In one aspect, the present disclosure provides a plant-based fermented product, the fermented product comprising gamma-glutamyl peptide (γ-glutamyl peptide) at a concentration of 700 ppm or more.

In an exemplary embodiment, the plant-based fermented product may comprise gamma-glutamyl peptide at a concentration of 700 ppm to 2000 ppm based on the weight of the fermented product. Specifically, the plant-based fermented product may comprise gamma-glutamyl peptide at a concentration of 700 ppm or more, 710 ppm or more, 720 ppm or more, 730 ppm or more, 740 ppm or more, 750 ppm or more, 760 ppm or more, 770 ppm or more, 780 ppm or more, 790 ppm or more, 800 ppm or more, 810 ppm or more, 820 ppm or more, 830 ppm or more, 840 ppm or more, 850 ppm or more, 860 ppm or more, 870 ppm or more, 880 ppm or more, 890 ppm or more, 900 ppm or more, 910 ppm or more, 920 ppm or more, 930 ppm or more, 940 ppm or more, 950 ppm or more, 960 ppm or more, 970 ppm or more, 980 ppm or more, 990 ppm or more, or more than 1000 ppm, and 2000 ppm or less, 1990 ppm or less, 1980 ppm less than, 1970 ppm or less, 1960 ppm or less, 1950 ppm or less, 1940 ppm or less, 1930 ppm or less, 1920 ppm or less, 1910 ppm or less, 1900 ppm or less, 1890 ppm or less, 1880 ppm or less, 1870 ppm or less, 1860 ppm or less, 1850 ppm or less, 1840 ppm or less, 1830 ppm or less, 1820 ppm or less, 1810 ppm or less, 1800 ppm or less, 1790 ppm or less, 1780 ppm or less, 1770 ppm or less, 1760 ppm or less, 1750 ppm or less, 1740 ppm or less, 1730 ppm or less, 1720 ppm or less, 1710 ppm or less, or 1700 ppm or less. The ppm may be based on the total weight of the plant-based fermented product.

In an exemplary embodiment, the gamma-glutamyl peptide may contain any one or more of the gamma-glutamyl dipeptide of γ-Glu-X and the gamma-glutamyl tripeptide of γ-Glu-X-Y.

In an exemplary embodiment, X and Y as described above may be the same or different amino acids. For example, the gamma-glutamyl tripeptide wherein X and Y are the same amino acids may be γ-Glu-Val-Val, γ-Glu-Glu-Glu, etc., and the gamma-glutamyl tripeptide wherein X and Y are different amino acids may be γ-Glu-Val-Cys, γ-Glu-Ala-Gly, etc.

In an exemplary embodiment, the amino acids may be selected from the group consisting of aspartic acid (Asp), threonine (Thr), serine (Ser), asparagine (Asn), glutamic acid (Glu), glutamine (Gln), cysteine (Cys), proline (Pro), glycine (Gly), alanine (Ala), valine (Val), methionine (Met), isoleucine (Ile), leucine (Leu), tyrosine (Tyr), phenylalanine (Phe), histidine (His), tryptophan (Trp), lysine (Lys), and arginine (Arg).

In an exemplary embodiment, the fermented product may be any one or more fermented products selected from the group consisting of fermented product of the plant-based materials, for example, soybeans, peas, chickpeas, lentils, Fava beans, mung beans, rice, and purified proteins thereof, but is not essentially limited thereto.

In an exemplary embodiment, the fermented product may be a fermented product of *Aspergillus oryzae* strain. The *Aspergillus oryzae* strain may be, for example, an *Aspergillus oryzae* SMF119 strain (deposit number KCTC12947BP).

In an exemplary embodiment, the fermented product may be a fermented product of *Bacillus amyloliquefaciens* strain. The *Bacillus amyloliquefaciens* strain may be, for example, a *Bacillus amyloliquefaciens* strain SMB469 (deposit number KCTC12822BP).

In an exemplary embodiment, the plant-based fermented product itself may be used as a flavoring material. Additionally, the plant-based fermented product may be processed to be used as the flavoring material, and the processing may include any one or more of mixing, separation, extraction, heating, sterilization, and drying.

In this specification, the flavoring material may refer to a substance that is added to a food product or is used in cooking to increase flavor and enhance taste. For example, the flavoring material may be soy sauce, red pepper paste, soybean paste, seasoned soy sauce, Kimchi sauce, Jangjorim sauce, Japchae sauce, pickle sauce, dressing sauce, tomato sauce, cream sauce, soup base, etc.

In one aspect, the present disclosure provides a food composition comprising the plant-based fermented product.

In an exemplary embodiment, the food product may include one or more selected from the group consisting of a low-salt food product, a plant-based food product, a meat replacement food product, a pet food product, an infant food product, and a health food product.

In this specification, the low-salt food product may mean a food product with reduced sodium content compared to an ordinary food product. For example, the low-salt food product may have a salinity of 1.5 % or less, 1.4 % or less, 1.3 % or less, 1.2 % or less, 1.1 % or less, 1 % or less, 0.95 % or less, 0.9 % or less, 0.85 % or less, 0.8 % or less, 0.75 % or less, 0.7 % or less, 0.65 % or less, 0.6 % or less, 0.55 % or less, 0.5 % or less, 0.49 % or less, 0.48 % or less, 0.47 % or less, 0.46 % or less, 0.45 % or less, 0.44 % or less, 0.43 % or less, 0.42 % or less, 0.41 % or less, 0.4 % or less, 0.39 % or less, 0.38 % or less, 0.37 % or less, 0.36 % or less, 0.35 % or less, 0.34 % or less, 0.33 % or less, 0.32 % or less, 0.31 % or less, 0.3 % or less, 0.29 % or less, 0.28 % or less, 0.27 % or less, 0.26 % or less, 0.25 % or less, 0.24 % or less, 0.23 % or less, 0.22 % or less, 0.21 % or less, 0.2 % or less, 0.19 % or less, 0.18 % or less, 0.17 % or less, 0.16 % or less, 0.15 % or less, 0.14 % or less, 0.13 % or less, 0.12 % or less, 0.11 % or less, or 0.1 % or less.

In this specification, the plant-based food product may refer to a food product in which animal-based materials such as meat, seafood, eggs, dairy products, etc. are replaced with the plant-based materials. For example, the plant-based food product may refer to a food product prepared or processed by replacing the animal-based material with the plant-based material.

In this specification, the meat replacement food product may refer to a food product made to replace meat such as beef, pork, and chicken to feel a flavor and texture similar to meat. An example of the meat replacement food product may include, but are not limited to, a plant-based meat replacement, a cell-cultured meat, and an insect protein food product.

In this specification, the pet food product may refer to a food product that includes all feeds such as staple foods and snacks consumed by the pets such as dogs and cats. For example, the pet food product may include a dry feed, a wet feed, a nutritious feed, a jerky for the pets, a dog gum, etc., but is not necessarily limited thereto.

In one aspect, the present disclosure provides a flavoring material containing the plant-based fermented product.

In an exemplary embodiment, the flavoring material may contain 0.001 to 99.99 % by weight of the plant-based fermented product, based on the total weight of the flavoring material. Specifically, the flavoring material may contain the plant-based fermented product in an amount of 0.001 % by weight or more, 0.002 % by weight or more, 0.003 % by weight or more, 0.004 % by weight or more, 0.005 % by weight or more, 0.006 % by weight or more, 0.007 % by weight or more, 0.008 % by weight or more, 0.009 % by weight or more, 0.01 % by weight or more, 0.02 % by weight or more, 0.03 % by weight or more, 0.04 % by weight or more, 0.05 % by weight or more, 0.1 % by weight or more, 0.2 % by weight or more, 0.3 % by weight or more, 0.4 % by weight or more, 0.5 % by weight or more, 1 % by weight or more, 2 % by weight or more, 3 % by weight or more, 4 % by weight or more, 5 % by weight or more, 6 % by weight or more, 7 % by weight or more, 8 % by weight or more, 9 % by weight or more, 10 % by weight or more, 11 % by weight or more, 12 % by weight or more, 13 % by weight or more, 14 % by weight or more, 15 % by weight or more, 16 % by weight or more, 17 % by weight or more, 18 % by weight or more, 19 % by weight or more, 20 % by weight or more, 21 % by weight or more, 22 % by weight or more, 23 % by weight or more, 24 % by weight or more, 25 % by weight or more, 26 % by weight or more, 27 % by weight or more, 28 % by weight or more, 29 % by weight or more, 30 % by weight or more, 31 % by weight or more, 32 % by weight or more, 33 % by weight or more, 34 % by weight or more, 35 % by weight or more, 36 % by weight or more, 37 % by weight or more, 38 % by weight or more, 39 % by weight or more, 40 % by weight or more, 41 % by weight or more, 42 % by weight or more, 43 % by weight or more, 44 % by weight or more, 45 % by weight or more, 46 % by weight or more, 47 % by weight or more, 48 % by weight or more, 49 % by weight or more, or 50 % by weight or more, and 99.99 % by weight or less, 99.98 % by weight or less, 99.97 % by weight or less, 99.96 % by weight or less, 99.95 % by weight or less, 99.94 % by weight or less, 99.93 % by weight or less, 99.92 % by weight or less, 99.91 % by weight or less, 99.9 % by weight or less, 99.8 % by weight or less, 99.7 % by weight or less, 99.6 % by weight or less, 99.5 % by weight or less, 99.4 % by weight or less, 99.3 % by weight or less, 99.2 % by weight or less, 99.1 % by weight or less, 99 % by weight or less, 98 % by weight or less, 97 % by weight or less, 96 % by weight or less, 95 % by weight or less, 94 % by weight or less, 93 % by weight or less, 92 % by weight or less, 91 % by weight or less, 90 % by weight or less, 89 % by weight or less, 88 % by weight or less, 87 % by weight or less, 86 % by weight or less, 85 % by weight or less, 84 % by weight or less, 83 % by weight or less, 82 % by weight or less, 81 % by weight or less, or 80 % by weight or less, based on the total weight of the flavoring material.

In an exemplary embodiment, in case the food product is cooked or prepared using the flavoring material, the flavoring material may be contained in an amount of 0.001 to 99.99 % by weight based on the total weight of the cooked or prepared food product, specifically in an amount of 0.001 % by weight or more, 0.002 % by weight or more, 0.003 % by weight or more, 0.004 % by weight or more, 0.005 % by weight or more, 0.006 % by weight or more, 0.007 % by weight or more, 0.008 % by weight or more, 0.009 % by weight or more, 0.01 % by weight or more, 0.02 % by weight or more, 0.03 % by weight or more, 0.04 % by weight or more, 0.05 % by weight or more, 0.1 % by weight or more, 0.2 % by weight or more, 0.3 % by weight or more, 0.4 % by weight or more, 0.5 % by weight or more, 1 % by weight or more, 1.5 % by weight or more, 2 % by weight or more, 3 % by weight or more, 4 % by weight or more, 5 % by weight or more, 6 % by weight or more, 7 % by weight or more, 8 % by weight or more, 9 % by weight or more, 10 % by weight or more, 11 % by weight or more, 12 % by weight or more, 13 % by weight or more, 14 % by weight or more, 15 % by weight or more, 16 % by weight or more, 17 % by weight or more, 18 % by weight or more, 19 % by weight or more, 20 % by weight or more, 21 % by weight or more, 22 % by weight or more, 23 % by weight or more, 24 % by weight or more, 25 % by weight or more, 26 % by weight or more, 27 % by weight or more, 28 % by weight or more, 29 % by weight or more, 30 % by weight or more, 31 % by weight or more, 32 % by weight or more, 33 % by weight or more, 34 % by weight or more, 35 % by weight or more, 36 % by weight or more, 37 % by weight or more, 38 % by weight or more, 39 % by weight or more, 40 % by weight or more, 41 % by weight or more, 42 % by weight or more, 43 % by weight or more, 44 % by weight or more, 45 % by weight or more, 46 % by weight or more, 47 % by weight or more, 48 % by weight or more, 49 % by weight or more, or 50 % by weight or more, and 99.99 % by weight or less, 99.98 % by weight or less, 99.97 % by weight or less, 99.96 % by weight or less, 99.95 % by weight or less, 99.94 % by weight or less, 99.93 % by weight or less, 99.92 % by weight or less, 99.91 % by weight or less, 99.9 % by weight or less, 99.8 % by weight or less, 99.7 % by weight or less, 99.6 % by weight or less, 99.5 % by weight or less, 99.4 % by weight or less, 99.3 % by weight or less, 99.2 % by weight or less, 99.1 % by weight or less, 99 % by weight or less, 98 % by weight or less, 97 % by weight or less, 96 % by weight or less, 95 % by weight or less, 94 % by weight or less, 93 % by weight or less, 92 % by weight or less, 91 % by weight or less, 90 % by weight or less, 89 % by weight or less, 88 % by weight or less, 87 % by weight or less, 86 % by weight or less, 85 % by weight or less, 84 % by weight or less, 83 % by weight or less, 82 % by weight or less, 81 % by weight or less, 80 % by weight or less, 79 % by weight or less, 78 % by weight or less, 77 % by weight or less, 76 % by weight or less, 75 % by weight or less, 74 % by weight or less, 73 % by weight or less, 72 % by weight or less, 71 % by weight or less, 70 % by weight or less, 60 % by weight or less, 50 % by weight or less, 40 % by weight or less, 30 % by weight or less, 20 % by weight or less, 10 % by weight or less, 5 % by weight or less, 4 % by weight or less, 3 % by weight or less, 2 % by weight or less, 1.5 % by weight or less, or 1 % by weight or less, based on the total weight of the cooked or prepared food product.

In an exemplary embodiment, the food product may contain at least 10 % by weight less sodium based on the total weight of the food product, compared to before addition of the flavoring material. Specifically, the food product may contain at least 10 % by weight less sodium, at least 11 % by weight less sodium, at least 12 % by weight less sodium, at least 13 % by weight less sodium, at least 14 % by weight less sodium, at least 15 % by weight less sodium, at least 16 % by weight less sodium, at least 17 % by weight less sodium, at least 18 % by weight less sodium, at least 19 % by weight less sodium, at least 20 % by weight less sodium, at least 21 % by weight less sodium, at least 22 % by weight less sodium, at least 23 % by weight less sodium, at least 24 % by weight less sodium, at least 25 % by weight less sodium, at least 26 % by weight less sodium, at least 27 % by weight less sodium, at least 28 % by weight less sodium, at least 29 % by weight less sodium, at least 30 % by weight less sodium, at least 40 % by weight less sodium, or at least 50 % by weight less sodium, based on the total weight of the food product, compared to before the addition of the flavoring material.

In an exemplary embodiment, in case the food product is cooked or prepared using the plant-based fermented product or the flavoring material, an amount of sodium used can be reduced by at least 10%, compared to the case where the plant-based fermented product or the flavoring material according to an embodiment of the present disclosure is not used. For example, in case the food product is cooked or prepared using the plant-based fermented product or the flavoring material, an amount of sodium used can be reduced by at least 10 % by weight, at least 11 % by weight, at least 12 % by weight, at least 13 % by weight, at least 14 % by weight, at least 15 % by weight, at least 16 % by weight, at least 17 % by weight, at least 18 % by weight at least, at least 19 % by weight, at least 20 % by weight, at least 21 % by weight, at least 22 % by weight, at least 23 % by weight, at least 24 % by weight, at least 25 % by weight, at least 26 % by weight, at least 27 % by weight, at least 28 % by weight, at least 29 % by weight, at least 30 % by weight, at least 31 % by weight, at least 32 % by weight, at least 33 % by weight, at least 34 % by weight, at least 35 % by weight, at least 36 % by weight, at least 37 % by weight, at least 38 % by weight, at least 39 % by weight, or at least 40 % by weight, compared to the case where the plant-based fermented product or the flavoring material according to an embodiment of the present disclosure is not used.

In an exemplary embodiment, the food composition may comprise a food auxiliary additive that is foodologically acceptable, and may further comprise an appropriate carrier, excipient, and diluent that are commonly used in the preparation of the food product.

In an exemplary embodiment, the food composition may further comprise, in addition to the above, various nutrients, vitamins, electrolytes, flavoring agents, colorants, pectic acid and its salts, alginic acid and its salts, organic acids, protective colloidal thickeners, pH adjusters, stabilizers, preservatives, glycerin, alcohol, carbonating agents used in carbonated drinks, etc.

In an exemplary embodiment, the plant-based fermented product contained in the food composition may be a plant-based fermented product of the processed form. For example, the processed form may mean being processed by any one or more of mixing, separation, extraction, heating, sterilization, and drying.

Among the processed forms, the mixing may refer to a state of mixing with other food additives in addition to the plant-based fermented product. Also, the separation among the processed forms may mean separating and purifying specific components of the plant-based fermented product using a membrane filtration technology, etc., and may refer to, for example, separating high molecular peptides and proteins to raise contents of low molecular peptides or amino acids in the plant-based fermented product.

In one aspect, the present disclosure provides a method for preparing a plant-based fermented product, comprising the steps of: inoculating and culturing *Aspergillus oryzae* strain with a plant-based material; and adding *Bacillus amyloliquefaciens* strain to the cultured plant-based material and fermenting it.

In an exemplary embodiment, the *Aspergillus oryzae* strain may be *Aspergillus oryzae* SMF119 strain. The *Aspergillus oryzae* SMF119 strain may have a deposit number of KCTC12947BP.

In an exemplary embodiment, the *Aspergillus oryzae* strain may be inoculated at a level of 1.0 x 10⁵ to 1.0 x 10⁸ CFU/g. Specifically, the *Aspergillus oryzae* strain may be inoculated at a level of 1.0 x 10⁵ CFU/g or more, 1.5 x 10⁵ CFU/g or more, 2.0 x 10⁵ CFU/g or more, 2.5 x 10⁵ CFU/g or more, 3.0 x 10⁵ CFU/g or more, 3.5 x 10⁵ CFU/g or more, 4.0 x 10⁵ CFU/g or more, 4.5 x 10⁵ CFU/g or more, or 5.0 x 10⁵ CFU/g or more, and 1.0 x 10⁸ CFU/g or less, 9.5 x 10⁷ CFU/g or less, 9.0 x 10⁷ CFU/g or less, 8.5 x 10⁷ CFU/g or less, 8.0 x 10⁷ CFU/g or less, 7.5 x 10⁷ CFU/g or less, 7.0 x 10⁷ CFU/g or less, 6.5 x 10⁷ CFU/g or less, 5.5 x 10⁷ CFU/g or less, or 5.0 x 10⁷ CFU/g or less.

In an exemplary embodiment, the step of inoculating and culturing the *Aspergillus oryzae* strain may be performed at 20 to 40°C for 40 to 72 hours. For example, the culturing step may be performed at 20°C or higher, 21°C or higher, 22°C or higher, 23°C or higher, 24°C or higher, or 25°C or higher, and at 40°C or lower, 39°C or lower, 38°C or lower, 37°C or lower, 36 °C or lower, or 35 °C or lower, for 40 hours or more, 42 hours or more, 44 hours or more, 46 hours or more, 48 hours or more, or 50 hours or more, and for 72 hours or less, 70 hours or less, 68 hours or less, 66 hours or less, 64 hours or less, 62 hours or less, or 60 hours or less.

In an exemplary embodiment, the *Bacillus amyloliquefaciens* strain may be a *Bacillus amyloliquefaciens* SMB469 strain. A deposit number of the *Bacillus amyloliquefaciens* SMB469 strain may be KCTC12822BP.

In an exemplary embodiment, the *Bacillus amyloliquefaciens* strain may be inoculated at a level of 1.0 x 10⁵ to 1.0 x 10⁸ CFU/g. Specifically, the *Bacillus amyloliquefaciens* strain may be inoculated at a level of 1.0 x 10⁵ CFU/g or more, 1.5 x 10⁵ CFU/g or more, 2.0 x 10⁵ CFU/g or more, 2.5 x 10⁵ CFU/g or more, 3.0 x 10⁵ CFU/g or more, 3.5 x 10⁵ CFU/g or more, 4.0 x 10⁵ CFU/g or more, 4.5 x 10⁵ CFU/g or more, or 5.0 x 10⁵ CFU/g or more, and 1.0 x 10⁸ CFU/g or less, 9.5 x 10⁷ CFU/g or less, 9.0 x 10⁷ CFU/g or less, 8.5 x 10⁷ CFU/g or less, 8.0 x 10⁷ CFU/g or less, 7.5 x 10⁷ CFU/g or less, 7.0 x 10⁷ CFU/g or less, 6.5 x 10⁷ CFU/g or less, 5.5 x 10⁷ CFU/g or less, or 5.0 x 10⁷ CFU/g or less.

In an exemplary embodiment, the fermenting step may be performed at 25 to 55°C for 5 to 200 days. For example, the fermentation step may be performed at 25°C or higher, 26°C or higher, 27°C or higher, 28°C or higher, 29°C or higher, or 30°C or higher, and at 55°C or lower, 54°C or lower, 53°C or lower, 52°C or lower, 51°C or lower, or 50°C or lower, for 5 days or more, 10 days or more, 15 days or more, 20 days or more, 25 days or more, or 30 days or more, and for 200 days or less, 195 days or less, 190 days or less, 185 days or less, 180 days or less, 175 days or less, 170 days or less, 165 days or less, 160 days or less, 165 days or less, or 160 days or less.

In an exemplary embodiment, the fermenting step may include adding brine to the cultured plant-based material, inoculating the *Bacillus amyloliquefaciens* strain, and performing the fermentation.

In an exemplary embodiment, the plant-based material may include any one or more selected from the group consisting of soybeans, peas, chickpeas, lentils, Fava beans, mung beans, rice, and purified proteins thereof.

In an exemplary embodiment, the preparing method may further comprise heat-treating the plant-based material before the culturing step. The heat treatment step denatures a protein of the plant-based material and kills microorganisms in the plant-based material.

In an exemplary embodiment, the heat treatment step may be performed at 100 to 130°C for 30 to 120 minutes. For example, the heat treatment step may be performed at 100°C or higher, 102°C or higher, 104°C or higher, 106°C or higher, 108°C or higher, or 110°C or higher, and at 130°C or lower, 128°C or lower, 126°C or lower, 124°C or lower, or 122°C or lower, for 30 minutes or more, 35 minutes or more, 40 minutes or more, 45 minutes or more, 50 minutes or more, or 55 minutes or more, and for 120 minutes or less, 115 minutes or less, 110 minutes or less, 105 minutes or less, 100 minutes or less, 95 minutes or less, 90 minutes or less.

In an exemplary embodiment, the heat treatment step may be performed under a pressure condition of 10 to 20l psi.

Hereinafter, the present disclosure will be described through Examples and the like in more detail. Since these Examples are intended only to illustrate the present disclosure, it will be obvious to those skilled in the art that the scope of the present disclosure should not be construed as being limited by these Examples.

### [Example 1] Preparation of plant-based fermented product

### (A) Step of heat treating a plant-based material

Purified water was added to soybeans and immersed at about 25°C for 1 hour to adjust moisture to 40 to 50%. Subsequently, the soy proteins were denatured and microorganisms were killed by heat treatment at a temperature of 121°C and a pressure of 151 psi for 1 hour.

### (B) Step of inoculating and cultivating Aspergillus oryzae strain

The heat-treated soybeans were cooled to 25°C and then inoculated with *Aspergillus oryzae* SMF119 strain (deposit number KCTC12947BP) at a level of 10⁶-10⁷ CFU/g and cultured at 30°C for 48 hours.

### (C) Step of fermenting cultured plant-based material with Bacillus amyloliquefaciens strain

Brine having a concentration of 20 to 30% (w/v) was added to the cultured plant-based material at 100 to 200% compared to the plant-based material, and was inoculated with *Bacillus amyloliquefaciens* SMB469 strain (deposit number KCTC12822BP) at a level of 10⁶ to 10⁷ CFU/g. Next, fermentation was performed for 180 days while maintaining a temperature of 30°C to prepare a plant-based fermented product. In this process, an enzymatic reaction is induced such that by the activity of leucine aminopeptidase, the proteins are hydrolyzed into glutamine, which is a substrate of gamma-glutamyltransferase, free amino acids and dipeptides, and that by the activity of gamma-glutamyltransferase, the gamma-glutamyl group of glutamine is transferred to free amino acids or dipeptides to produce gamma-glutamyl peptide.

### [Example 2]

A plant-based fermented product was prepared in the same method as that of Example 1, except that *Aspergillus oryzae* RIB40 (NBRC 100959), a standard Aspergillus oryzae strain, was used instead of the *Aspergillus oryzae* SMF119 strain.

### [Example 3]

A plant-based fermented product was prepared in the same method as that of Example 1, except that rice was used instead of the soybeans.

### [Example 4]

A plant-based fermented product was prepared in the same method as that of Example 2, except that rice was used instead of the soybeans.

### [Example 5]

A plant-based fermented product was prepared in the same method as that of Example 1, except that pea proteins were used instead of the soybeans.

### [Example 6] Preparation of flavoring material

The plant-based fermented product prepared by Example 5 was filtered through a filter press to separate and remove high molecular components and improve concentrations of low molecular peptides and amino acids including gamma-glutamyl peptide, and was prepared a liquid form so as to facilitate the subsequent processing steps. This plant-based fermented product was sterilized at 70 to 120°C for 5 seconds to 24 hours and then spray-dried to prepare a powdery flavoring material. When applied to cooking or a food product, the flavoring material has effects of increasing a taste and enhancing a flavor of food materials, thereby achieving an effect of reducing sodium intake (salt reduction).

### [Comparative Example 1]

Purified water was added to soybeans and immersed at about 25°C for 1 hour to adjust a moisture content to 40 to 50%. Thereafter, the soybean proteins were denatured and microorganisms were killed by heat treatment at a temperature of 121°C and a pressure of 151 psi for 1 hour. The heat-treated soybeans were cooled to 25°C and then inoculated with *Aspergillus oryzae* SMF119 strain (deposit number KCTC12947BP) at a level of 10⁶-10⁷ CFU/g and cultured at 30°C for 48 hours. Comparative Example 1) was prepared by adding 100 to 200% of brine compared to the cultured plant-based material, at a concentration of 20 to 30% (w/v), to the cultured plant-based material, and fermenting for 180 days while maintaining a temperature at 30°C.

### [Comparative Example 2]

Comparative Example 2 was prepared in the same method as that of Comparative Example 1, except that *Aspergillus oryzae* RIB40 (NBRC 100959), a standard *Aspergillus oryzae* strain, was used instead of the *Aspergillus oryzae* SMF119 strain.

### [Comparative Example 3]

Comparative Example 3 was prepared in the same method as that of Comparative Example 1, except that rice was used instead of the soybeans.

### [Comparative Example 4]

Comparative Example 4 was prepared in the same method as that of Comparative Example 2, except that rice was used instead of the soybeans.

### [Experimental Example 1] Confirmation of gamma-glutamyl peptide content

Examples 1 to 4 and Comparative Examples 1 to 4 were filtered with a whatman No.2 filter paper, and gamma-glutamyl peptide contents of the filtrates were measured. The gamma-glutamyl peptide contents were measured three times repeatedly using HPLC-MSMS (OSAKA SODA NANOSPACE SI-2, AB SCIEX Triple Quad 5500+), and the results were shown in Tables 1 and 2.

**[Table 1]**

| (Unit: ppm) | Example 1 | Example 2 | Comparative Example 1 | Comparative Example 2 |
|---|---|---|---|---|
| γ-Glu-His | 142.8 | 91.9 | 26.7 | 26.7 |
| γ-Glu-Leu | 72.7 | 35.6 | 22.6 | 22.6 |
| γ-Glu-Lys | 49.4 | 34.9 | 17.3 | 17.3 |
| EVG (γ-Glu-val-Gly) | 0.0 | 0.0 | 7.7 | 7.7 |
| EV(γ-Glu-val) | 62.9 | 33.6 | 40.3 | 40.3 |
| γ-Glu-Tyr | 35.2 | 17.0 | 72.1 | 72.1 |
| γ-Glu-Phe | 88.5 | 44.7 | 43.8 | 43.8 |
| γ-Glu-Arg | 34.4 | 14.2 | 12.0 | 12.0 |
| γ-Glu-Thr | 130.1 | 102.3 | 40.8 | 40.8 |
| γ-Glu-Ile | 64.9 | 35.6 | 18.8 | 18.8 |
| γ-Glu-Glu | 270.1 | 93.1 | 73.3 | 73.3 |
| γ-Glu-Trp | 5.5 | <15 ug/kg | 0.9 | 0.9 |
| γ-Glu-Gly | 115.2 | 31.5 | 15.4 | 15.4 |
| γ-Glu-Asn | 25.7 | 0.0 | 0.0 | 0.0 |
| γ-Glu-Ser | 207.7 | 120.7 | 66.5 | 66.5 |
| γ-Glu-Met | 45.3 | 20.0 | 28.7 | 28.7 |
| γ-Glu-Ala | 38.7 | 19.0 | 15.6 | 15.6 |
| γ-Glu-Orn | 32.8 | 36.1 | 0.0 | 0.0 |
| γ-Glu-val_ol | 0.0 | <10.0 ug/kg | <10.0 ug/kg | <10.0 ug/kg |
| γ-Glu-Cys | 4.6 | 2.0 | <25.0 ug/kg | <25.0 ug/kg |
| γ-Glu-Pro | 0.0 | 0.0 | 0.0 | 0.0 |
| γ-Glu-Ile-Gly | 0.0 | 0.0 | 0.0 | 0.0 |
| γ-Glu-Val-Cys | 0.0 | 0.0 | 0.0 | 0.0 |
| γ-Glu-Val-Gln | 0.0 | 0.0 | 0.0 | 0.0 |
| γ-Glu-Gln | 9.2 | <15 ug/kg | 9.3 | 9.3 |
| γ-Glu-Ala-Gly | 0.0 | 0.0 | 0.0 | 0.0 |
| γ-GSH-reduced | 0.0 | 0.0 | 0.0 | 0.0 |
| γ-Glu-Glu-Glu | 0.0 | <10.0 ug/kg | <10.0 ug/kg | <10.0 ug/kg |
| γ-Glu-Ser-Gly | 30.7 | 8.8 | 13.6 | 13.6 |
| γ-Glu-Asp | 0.0 | 0.0 | 0.0 | 0.0 |
| Sum | 1466.0 | 741.0 | 525.5 | 316.8 |

**[Table 2]**

| (Unit: ppm) | Example 3 | Example 4 | Comparative Example 3 | Comparative Example 4 |
|---|---|---|---|---|
| γ-Glu-His | 188.9 | 87.6 | 58.1 | 51.5 |
| γ-Glu-Leu | 90.5 | 43.0 | 31.1 | 29.4 |
| γ-Glu-Lys | 118.1 | 67.7 | 46.5 | 38.1 |
| EVG (γ-Glu-val-Gly) | 0.0 | 6.0 | 3.7 | 0.0 |
| EV(γ-Glu-val) | 88.9 | 69.0 | 46.9 | 30.4 |
| γ-Glu-Tyr | 82.2 | 15.1 | 10.2 | 24.6 |
| γ-Glu-Phe | 83.8 | 42.4 | 27.5 | 29.0 |
| γ-Glu-Arg | 9.8 | 46.5 | 34.5 | 1.3 |
| γ-Glu-Thr | 354.9 | 64.2 | 46.3 | 90.8 |
| γ-Glu-Ile | 98.8 | 39.5 | 31.0 | 19.2 |
| γ-Glu-Glu | 293.4 | 193.4 | 136.1 | 75.3 |
| γ-Glu-Trp | <15 ug/kg | 2.4 | 1.7 | <15 ug/kg |
| γ-Glu-Gly | 130.4 | 69.0 | 55.1 | 30.7 |
| γ-Glu-Asn | 0.0 | 0.0 | 0.0 | 0.0 |
| γ-Glu-Ser | 228.8 | 129.2 | 89.8 | 54.0 |
| γ-Glu-Met | 93.1 | 17.4 | 11.9 | 22.5 |
| γ-Glu-Ala | 27.9 | 47.3 | 33.4 | 25.6 |
| γ-Glu-Orn | 0.0 | 30.8 | 19.1 | 14.3 |
| γ-Glu-val_ol | 0.0 | 0.0 | 0.0 | 0.0 |
| γ-Glu-Cys | 4.9 | 2.7 | 1.8 | 1.6 |
| γ-Glu-Pro | 0.0 | 0.0 | 0.0 | 0.0 |
| γ-Glu-Ile-Gly | 0.0 | 0.0 | 0.0 | 0.0 |
| γ-Glu-Val-Cys | 0.0 | 0.0 | 0.0 | 0.0 |
| γ-Glu-Val-Gln | 0.0 | 0.0 | 0.0 | 0.0 |
| γ-Glu-Gln | 0.0 | 11.6 | 8.2 | 0.0 |
| γ-Glu-Ala-Gly | 0.0 | 0.0 | 0.0 | 0.0 |
| γ-GSH-reduced | 14.3 | 0.0 | 0.0 | 11.4 |
| γ-Glu-Glu-Glu | 6.6 | <15 ug/kg | <15 ug/kg | 0.0 |
| γ-Glu-Ser-Gly | 35.6 | 19.8 | 13.1 | 22.1 |
| γ-Glu-Asp | 188.4 | 138.8 | 97.0 | 53.5 |
| Sum | 2,139.3 | 1,143.4 | 803.0 | 625.3 |

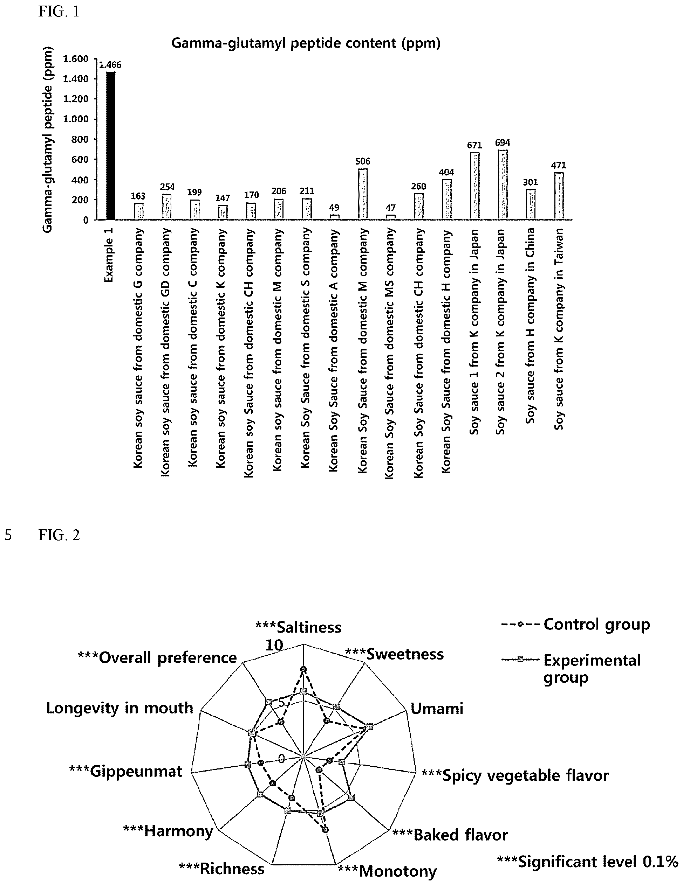

Further, gamma-glutamyl peptide contents of the domestic traditional soy sauce products that are made from soybeans as a raw material and subjected to fermentation period for 1 to 10 years and the brewed soy sauce products that are sold overseas using wheat as the raw material were measured three times in the same method as above, and the results were shown in Tables 3 to 5 and compared with Example 1 in FIG. 1.

**[Table 3]**

| (Unit: ppm) | Korean soy sauce from domestic G company | Korean soy sauce from domestic GD company | Korean soy sauce from domestic C company | Korean soy sauce from domestic K company | Korean soy Sauce from domestic CH company | Korean soy sauce from domestic M company |
|---|---|---|---|---|---|---|
| γ-Glu-His | 15.56 | 25.87 | 11.63 | 14.94 | 2.16 | 19 |
| γ-Glu-Leu | 6.78 | 8.8 | 5.97 | 4.99 | 8.67 | 8.8 |
| γ-Glu-Lys | 15.22 | 18.55 | 10.14 | 10.3 | 14.05 | 17.32 |
| EVG (γ-Glu-val-Gly) | 0 | 0 | 0 | 0 | 0 | 0 |
| EV(γ-Glu-val) | 8.57 | 11.77 | 11.05 | 6.27 | 10.33 | 9.66 |
| γ-Glu-Tyr | 8.28 | 10.64 | 23.52 | 20.76 | 9.84 | 10.25 |
| γ-Glu-Phe | 13.57 | 12.76 | 23.28 | 18.47 | 16.01 | 10.83 |
| γ-Glu-Arg | 0 | 0.93 | 4.8 | 1.94 | 5.29 | 1.56 |
| γ-Glu-Thr | 13.31 | 28.48 | 11 | 11.34 | 18.85 | 24.55 |
| γ-Glu-Ile | 7.11 | 9.46 | 6.43 | 4 | 9.23 | 9 |
| γ-Glu-Glu | 36.93 | 57.05 | 34.62 | 26.99 | 49.21 | 49.06 |
| γ-Glu-Trp | 0 | <15.0 ug/kg | <15.0 ug/kg | <15.0 ug/kg | <15.0 ug/kg | 0 |
| γ-Glu-Gly | 13.79 | 31.22 | 14.52 | 8.62 | 0 | 11.81 |
| γ-Glu-Asn | 0 | 0 | 0 | 0 | 0 | 0 |
| γ-Glu-Ser | 10.43 | 25.91 | 11.03 | 12.93 | 17.48 | 16.52 |
| γ-Glu-Met | 0 | 2.51 | 0.95 | < 5.0 ug/kg | < 5.0 ug/kg | 0.33 |
| γ-Glu-Ala | 6.15 | 5.85 | 8.28 | 2.85 | 4.55 | 5.65 |
| γ-Glu-Orn | 6.99 | 0 | 4.3 | 0 | 0 | 0 |
| γ-Glu-val_ol | 0 | 0 | 0 | 0 | 0 | 0 |
| γ-Glu-Cys | 0 | 0 | 0 | 0 | 0.36 | 0.57 |
| γ-Glu-Pro | 0 | < 2.5 ug/kg | 0 | 0 | 0 | 0 |
| γ-Glu-Ile-Gly | 0 | 1.13 | 0 | 0.93 | 0 | 2.24 |
| γ-Glu-Val-Cys | 0 | 0 | 0 | 0 | 0 | 0 |
| γ-Glu-Val-Gln | 0 | 0 | 0 | 0 | 0 | 0 |
| γ-Glu-Gln | 0 | 0 | 1.54 | 1.26 | 0.43 | <15.0 ug/kg |
| γ-Glu-Ala-Gly | 0 | 0 | 0 | 0 | 0 | 0 |
| γ-GSH-reduced | 0 | 0 | 0 | 0 | 0 | 0 |
| γ-Glu-Glu-Glu | 0 | 0 | 14.85 | <10.0 ug/kg | 3.69 | 7.22 |
| γ-Glu-Ser-Gly | 0.38 | 3.11 | 1.34 | 0.25 | < 2.5 ug/kg | 1.46 |
| γ-Glu-Asp | 0 | 0 | 0 | 0 | 0 | 0 |
| Total (ppm) | 163.1 | 254.0 | 199.3 | 146.8 | 170.2 | 205.8 |

**[Table 4]**

| (Unit: ppm) | Korean Soy Sauce from domestic S company | Korean Soy Sauce from domestic A company | Korean Soy Sauce from domestic M company | Korean Soy Sauce from domestic MS company | Korean Soy Sauce from domestic CH company | Korean Soy Sauce from domestic H company |
|---|---|---|---|---|---|---|
| γ-Glu-His | 24.51 | 4.55 | 20.37 | 2.08 | 33.36 | 53.11 |
| γ-Glu-Leu | 10.23 | 2.95 | 21.5 | 0 | 10.31 | 16.6 |
| γ-Glu-Lys | 18.39 | 8.54 | 23.91 | 2.26 | 21.46 | 26.6 |
| EVG | 0 | 0 | 0 | 0 | 0 | 1.51 |
| EV(γ-Glu-val) | 13.28 | 4.29 | 40.43 | < 5.0 ug/kg | 13.96 | 23.15 |
| γ-Glu-Tyr | 13.01 | 2.3 | 23.65 | 15.94 | 17.07 | 23.41 |
| γ-Glu-Phe | 17.58 | 4.53 | 27.27 | 15.44 | 21.24 | 37.28 |
| γ-Glu-Arg | 2.2 | 0 | 14.49 | 1.58 | 13.85 | 5.53 |
| γ-Glu-Thr | 20.52 | 4.04 | 49.21 | < 5.0 ug/kg | 18.43 | 29.19 |
| γ-Glu-Ile | 7.53 | 3.22 | 21.17 | < 5.0 ug/kg | 10.6 | 14.78 |
| γ-Glu-Glu | 43.74 | 8.34 | 112.24 | 3.4 | 41.89 | 72.94 |
| γ-Glu-Trp | 0 | <15.0 ug/kg | <15.0 ug/kg | 0 | <15.0 ug/kg | 0 |
| γ-Glu-Gly | 0 | 1.74 | 34.83 | 1.96 | 24.49 | 36.77 |
| γ-Glu-Asn | 0 | 0 | 0 | 0 | 0 | 0 |
| γ-Glu-Ser | 19.41 | 1.41 | 60.58 | <10.0 ug/kg | 18.51 | 35.15 |
| γ-Glu-Met | 1.35 | 0.94 | 19.8 | 0 | 1.61 | < 5.0 ug/kg |
| γ-Glu-Ala | 4.73 | 1.42 | 22.47 | <20.0 ug/kg | 6.92 | 17.92 |
| γ-Glu-Orn | 10.21 | 0 | 0 | 0 | 0 | 0 |
| γ-Glu-val_ol | 0 | 0 | 0 | 0 | 0 | 0 |
| γ-Glu-Cys | 0.58 | 0 | 0 | 0 | 0 | 0 |
| γ-Glu-Pro | 0 | < 2.5 ug/kg | < 2.5 ug/kg | 0 | 0 | 0 |
| γ-Glu-Ile-Gly | 1.81 | 0.42 | 2.77 | 0 | 0 | 2.4 |
| γ-Glu-Val-Cys | 0 | 0 | 0 | 0 | 0 | 0 |
| γ-Glu-Val-Gln | 0 | 0 | 0 | 0 | 0 | 0 |
| γ-Glu-Gln | 1.01 | 0 | 3.27 | <15.0 ug/kg | 5.36 | 1.41 |
| γ-Glu-Ala-Gly | 0 | 0 | 0 | 0 | 0 | 0 |
| γ-GSH-reduced | 0 | 0 | 0 | 0 | 0 | 0 |
| γ-Glu-Glu-Glu | 0 | 0 | 4.69 | 4.55 | 0 | 4.55 |
| γ-Glu-Ser-Gly | 1.24 | 0 | 3.33 | 0 | 0.49 | 1.91 |
| γ-Glu-Asp | 0 | 0 | 0 | 0 | 0 | 0 |
| Sum | 211.3 | 48.7 | 506.0 | 47.2 | 259.6 | 404.2 |

**[Table 5]**

| (Unit: ppm) | Soy sauce 1 from K company in Japan | Soy sauce 2 from K company in Japan | Soy sauce from H company in China | Soy sauce from K company in Taiwan |
|---|---|---|---|---|
| γ-Glu-His | 30.49 | 38.68 | 15.2 | 27.29 |
| γ-Glu-Leu | 44.13 | 39.39 | 19.21 | 30.07 |
| γ-Glu-Lys | 31.12 | 40.06 | 12.19 | 24.15 |
| EVG | 3.23 | 1.88 | 0 | 2.8 |
| EV(γ-Glu-val) | 60.99 | 59.2 | 27.77 | 45.34 |
| γ-Glu-Tyr | 17.53 | 22.09 | 11.55 | 17.74 |
| γ-Glu-Phe | 55.26 | 56.34 | 26.29 | 34.28 |
| γ-Glu-Arg | 42.48 | 57.41 | 7.32 | 30.74 |
| γ-Glu-Thr | 29.42 | 54.64 | 15.47 | 30.65 |
| γ-Glu-Ile | 45.6 | 36.53 | 18.38 | 28.54 |
| γ-Glu-Glu | 143.07 | 107.67 | 60.56 | 68.82 |
| γ-Glu-Trp | <15.0 ug/kg | <15.0 ug/kg | <15.0 ug/kg | <15.0 ug/kg |
| γ-Glu-Gly | 30.14 | 44.31 | 10.72 | 20.52 |
| γ-Glu-Asn | 0 | 0 | 0 | 0 |
| γ-Glu-Ser | 57.5 | 89.7 | 37.24 | 54.8 |
| γ-Glu-Met | 25.8 | 17.26 | 12.6 | 20.61 |
| γ-Glu-Ala | 41.22 | 26.52 | 14.85 | 20.47 |
| γ-Glu-Orn | 0 | 0 | 2.58 | 0 |
| γ-Glu-val_ol | 0 | 0 | 0 | 0 |
| γ-Glu-Cys | 0 | 0 | 0 | 0.47 |
| γ-Glu-Pro | 0 | 0 | 0 | 0 |
| γ-Glu-Ile-Gly | 2.25 | 0.77 | 0.28 | 1.59 |
| γ-Glu-Val-Cys | 0 | 0 | 0 | 0 |
| γ-Glu-Val-Gln | 0 | 0 | 0 | 0 |
| γ-Glu-Gln | 5.96 | 0 | 8.87 | 10.18 |
| γ-Glu-Ala-Gly | 0 | 0 | 0 | 0 |
| γ-GSH-reduced | 0 | 0 | 0 | 0 |
| γ-Glu-Glu-Glu | 0 | 0 | 0 | 0 |
| γ-Glu-Ser-Gly | 4.95 | 1.94 | 0.28 | 1.49 |
| γ-Glu-Asp | 0 | 0 | 0 | 0 |
| Sum | 671.1 | 694.4 | 301.4 | 470.6 |

As shown in Table 1, it could be confirmed that Examples 1 and 2 in which the soybeans, the plant-based material, were fermented with *Bacillus amyloliquefaciens* SMB469 having excellent gamma-glutamyltransferase activity showed high gamma-glutamyl peptide contents, and especially, Example 1 which used *Aspergillus oryzae* SMF119 having excellent leucine aminopeptidase activity showed gamma-glutamyl peptide contents greatly improved to 1,450 ppm or more. In addition, as shown in Table 2, it could be confirmed that Examples 3 and 4 in which the rice, the plant-based material, was fermented with *Bacillus amyloliquefaciens* SMB469 having excellent gamma-glutamyltransferase activity showed high gamma-glutamyl peptide contents, and especially, Example 3 which used *Aspergillus oryzae* SMF119 having excellent leucine aminopeptidase activity showed gamma-glutamyl peptide contents greatly improved to 2,100 ppm or more. Through these results, it can be seen that the method for preparing the plant-based fermented product according to an embodiment of the present disclosure which used *Aspergillus oryzae* SMF119 having excellent leucine aminopeptidase activity and/or *Bacillus amyloliquefaciens* SMB469 having excellent gamma-glutamyltransferase activity can be extended to protein raw materials composed of 20 types of amino acids, including glutamine, a gamma-glutamyl group donor substrate, such as peas, chickpeas, lentils, Fava beans, mung beans, and purified proteins thereof, in addition to the soybeans and the rice, and that this preparation method enables to obtain the plant-based fermented product having enhanced gamma-glutamyl peptide contents.

Further, as shown in Tables 3 to 5, it could be confirmed that gamma-glutamyl peptide contents of the domestic soy sauce products made by using the soybeans as the raw material and fermented for 1 to 10 years and the oversea soy sauce products made by using as the raw material the wheat having a high content of glutamine, the gamma-glutamyl group donor substrate, were very low at a level of 4 to 50%, compared to the gamma-glutamyl peptide contents of the plant-based fermented product of the present disclosure. Through this, it could be seen that the gamma-glutamyl peptide of high contents cannot be produced simply by lengthening the fermentation period or increasing contents of the substrate.

### [Experimental Example 2] Evaluation of sensory characteristics of plant-based fermented product

In order to confirm an effect exerted by applying the plant-based fermented product of the present disclosure to a food product, sensory characteristics were evaluated as follows. Samples made by boiling a beef radish soup, taking out the dry matters therefrom, adding 21g of each of Example 1 and Comparative Example 1 to 500g of the soup, and boiling it for 1 minute, were evaluated on sensory characteristics (15-point scale, 2 repetitions) by 9 trained professional panels. Regarding the sensory characteristic evaluation results, significant differences between the samples were verified at a significance level of 5% using a paired sample t test. The results were shown in Table 6.

**[Table 6]**

| | Sweetness | Saltiness | Umami | Beef flavor | Richness | Harmony | Gippeunmat |
|---|---|---|---|---|---|---|---|
| Example 1 | 5.6^{a} | 6.8^{a} | 6.1^{a} | 6.6^{a} | 5.9^{a} | 5.8^{a} | 5.8^{a} |
| Comparative Example 1 | 4.2^{b} | 5.6^{b} | 4.9^{b} | 5.2^{b} | 4.6^{b} | 4.7^{b} | 4.7^{b} |
| p-value | 0.00 | 0.00 | 0.00 | 0.00 | 0.00 | 0.00 | 0.00 |

As shown in Table 6, Example 1, which contained gamma-glutamyl peptide contents of 1,450 ppm or more, significantly improved the sweetness, saltiness, umami, beef flavor, richness, harmony, and Gippeunmat attribute strength for the beef radish soup. Accordingly, it could be confirmed that when the plant-based fermented product of the present disclosure comprising a high content of gamma-glutamyl peptide is used in the food product, it has excellent usability as a flavoring material improving deep and rich flavors.

### [Experimental Example 3] Confirmation of gamma-glutamyl peptide content of flavoring material

A gamma-glutamyl peptide content of the flavoring material of Example 6 was measured three times repeatedly using HPLC-MSMS (OSAKA SODA NANOSPACE SI-2, AB SCIEX Triple Quad 5500+), and the results were shown in Table 7.

**[Table 7]**

| (Unit: ppm) | Example 4 |
|---|---|
| γ-Glu-His | 222.6 |
| γ-Glu-Leu | 280.6 |
| γ-Glu-Lys | 295.8 |
| EVG(γ-Glu-val-Gly) | 41.2 |
| EV(γ-Glu-val) | 435.4 |
| γ-Glu-Tyr | 46.0 |
| γ-Glu-Phe | 245.6 |
| γ-Glu-Arg | 378.8 |
| γ-Glu-Thr | 538.0 |
| γ-Glu-Ile | 271.4 |
| γ-Glu-Glu | 930.4 |
| γ-Glu-Trp | <15 ug/kg |
| γ-Glu-Gly | 295.8 |
| γ-Glu-Asn | 0.0 |
| γ-Glu-Ser | 886.2 |
| γ-Glu-Met | 147.4 |
| γ-Glu-Ala | 331.9 |
| γ-Glu-Orn | 0.0 |
| γ-Glu-val_ol | 0.0 |
| γ-Glu-Cys | <25 ug/kg |
| γ-Glu-Pro | 0.0 |
| γ-Glu-Ile-Gly | 34.0 |
| γ-Glu-Val-Cys | 0.0 |
| γ-Glu-Val-Gln | 0.0 |
| γ-Glu-Gln | 93.6 |
| γ-Glu-Ala-Gly | 0.0 |
| γ-GSH-reduced | 0.0 |
| γ-Glu-Glu-Glu | 1.6 |
| γ-Glu-Ser-Gly | 195.3 |
| γ-Glu-Asp | 842.0 |
| Sum | 6,513.6 |

As can be seen from Table 7, it could be confirmed that the content of gamma-glutamyl peptide of Example 6 was very high at 6,500 ppm or more.

### [Experimental Example 4] Evaluation of effect of applying plant-based fermented product

In order to confirm an effect of applying the flavoring material according to an example of the present disclosure to a food product, sensory characteristics were evaluated as follows. After an olive oil was put into a pot, onions, carrots and celery were added to the pot, and fried over low heat for 2 minutes. Then, water was poured into the pot to boil it over high heat. When water starts to boil, it was lowered to low heat and boiled for about 20 minutes, and only the clear liquid obtained by filtration through a sieve was used as a plant-based consomme soup. 0.3-1.68 % (w/w) of Example 6 was added to the plant-based consomme soup, a refined salt was added to adjust a salinity to 0.8%, and then the sensory characteristics (15-point scale) were evaluated by 10 trained professional panels and the results were shown in Table 8.

**[Table 8]**

| | Sweetness | Saltiness | Umami | Longevity in mouth | Richness | Degree of desirability |
|---|---|---|---|---|---|---|
| Example 6 0.3%(w/w) | 4.9 | 4.8 | 5.3 | 5.4 | 4.7 | 5.2 |
| Example 6 0.5%(w/w) | 5.4 | 5.7 | 6.7 | 6.7 | 6.7 | 6.9 |
| Example 6 1%(w/w) | 6.9 | 6.6 | 7.4 | 7.7 | 7.7 | 7.4 |
| Example 6 1.68%(w/w) | 8.3 | 8.1 | 9.0 | 9.1 | 7.2 | 4.2 |

### [Experimental Example 5] Evaluation of effect of reducing sodium intake

In order to evaluate an effect of reducing sodium intake by the plant-based fermented product according to an example of the present disclosure, sensory characteristics were evaluated (15-point scale, 2 repetitions) by 15 trained professional panels on a control group in which only refined salt was added into the plant-based consomme soup prepared in the same method as that of Experimental Example 4 to adjust the salinity to 0.8 % (w/w) and an experimental group in which 1 % (w/w) of Example 6 was added into the plant-based consomme soup to adjust the salinity to 0.56 % (w/w) with the refined salt. Regarding the sensory characteristic evaluation results, significant differences between the samples were verified at a significance level of 5% using a paired sample t test, and the results were shown in Table 9 and FIG. 2.

**[Table 9]**

| | Salti ness | Sweet ness | Uma mi | Spicy vegetab le flavor | Baked flavor | Monot ony | Rich ness | Harm ony | Gippeu nmat | Longevity in mouth | Overall preferen ce |
|---|---|---|---|---|---|---|---|---|---|---|---|
| Control group | 7.8 ^{a} | 3.8 ^{b} | 6.0 | 2.3 ^{b} | 1.8 ^{b} | 6.8 ^{a} | 3.8 ^{b} | 3.6 ^{b} | 3.8 ^{b} | 4.9 | 3.7 ^{b} |
| Experi mental group | 5.8 ^{b} | 5.3 ^{a} | 6.4 | 3.4 ^{a} | 5.6 ^{a} | 5.3 ^{b} | 5.0 ^{a} | 5.1 ^{a} | 5.0 ^{a} | 5.1 | 5.8 ^{a} |
| *p*-value | 0.00 | 0.00 | 0.46 | 0.00 | 0.00 | 0.00 | 0.00 | 0.00 | 0.00 | 0.81 | 0.00 |

As shown in Table 9 and FIG. 2, it was confirmed that the soup prepared by Example 6 containing a high content of gamma-glutamyl peptide enhanced the spicy vegetable flavor, the baked flavor (savory flavor felt when potatoes, etc. are baked), the richness (a degree to which various flavor characteristics are felt in a complex manner in addition to the basic taste), harmony (a degree to which an overall balance is felt without any one characteristic standing out), and the Gippeunmat (a degree to which the ingredients are thoroughly boiled so that a flavor of the soup is felt in depth), and decreased the monotony (a degree in which a taste is felt monotonous due to a small number of flavor characteristics), whereby the overall preference of the soup prepared by Example 6 was increased compared to the control group with high salinity.

### [Deposit Number]

*Aspergillus oryzae* SMF119 strain
Name of depository institution: Korea Research Institute of Bioscience and Biotechnology
Deposit number: KCTC12947BP
Trust date: 20151112
*Bacillus amyloliquefaciens* SMB-469 strain
Name of depository institution: Korea Research Institute of Bioscience and Biotechnology
Deposit number: KCTC12822BP
Trust date: 20150529

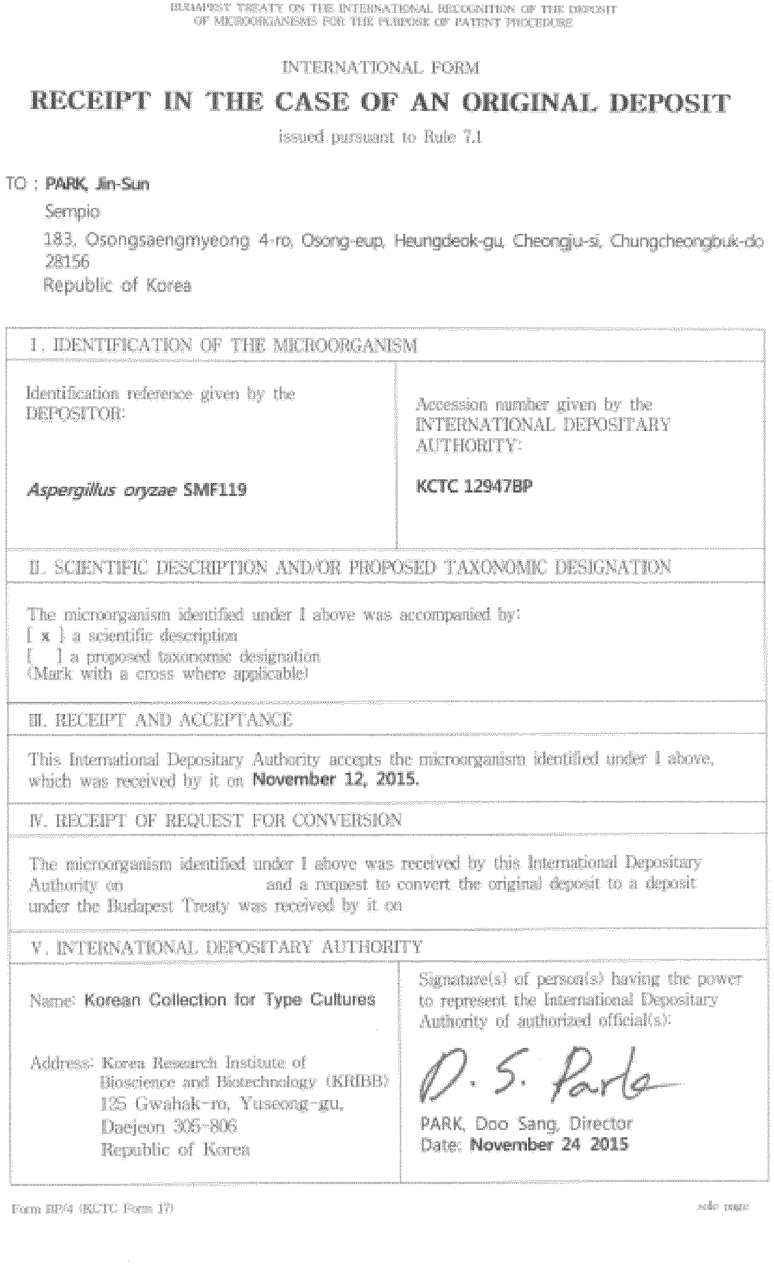

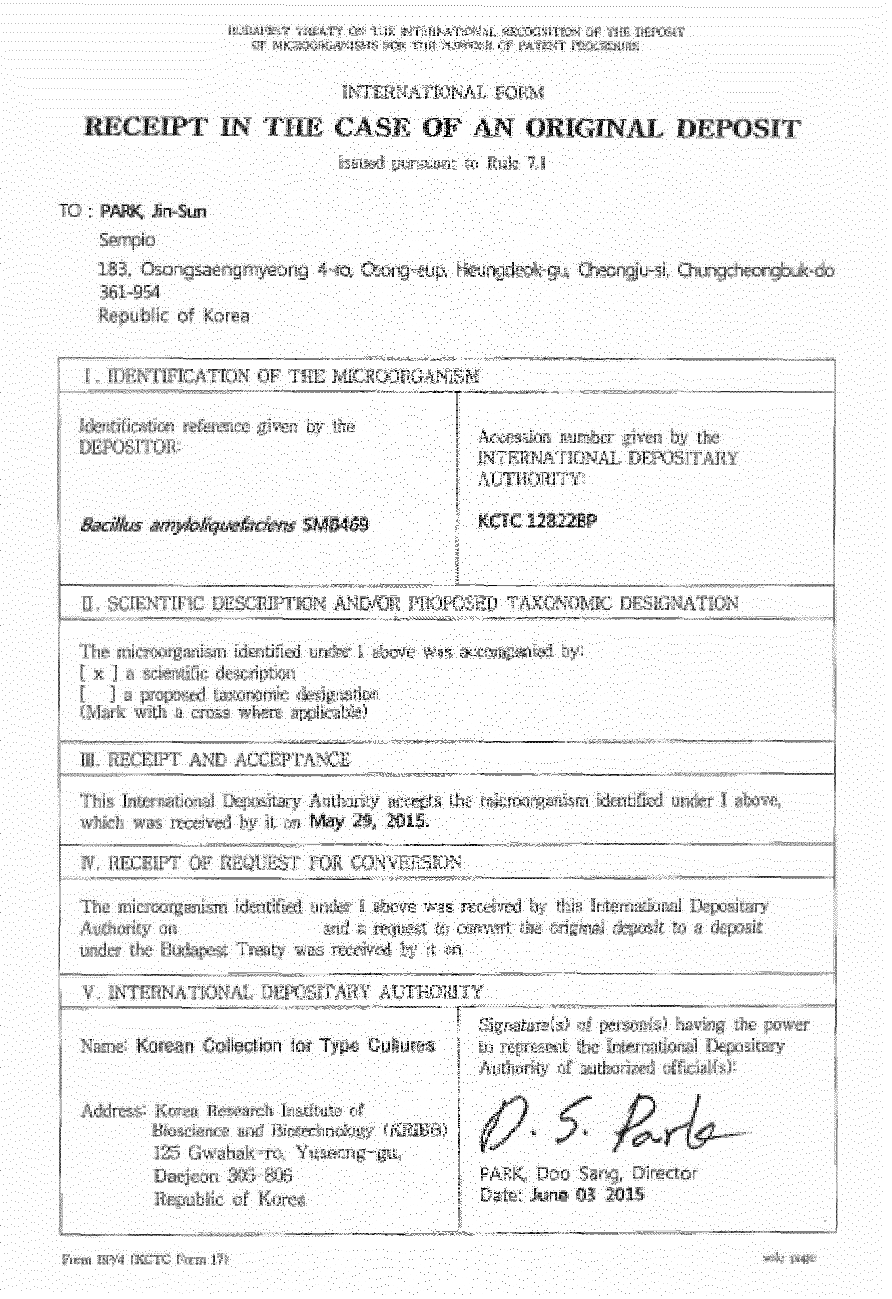

## Claims

1. A plant-based fermented product, wherein the fermented product comprises gamma-glutamyl peptide at a concentration of 700 ppm or more based on the weight of the fermented product.

2. The plant-based fermented product according to claim 1, wherein the gamma-glutamyl peptide comprises at least one of gamma-glutamyl dipeptide of γ-Glu-X and gamma-glutamyl tripeptide of γ-Glu-X-Y.

3. The plant-based fermented product according to claim 2, wherein X and Y are the same or different amino acids, and wherein the amino acids are selected from the group consisting of aspartic acid (Asp), threonine (Thr), serine (Ser), asparagine (Asn), glutamic acid (Glu), glutamine (Gln), cysteine (Cys), proline (Pro), glycine (Gly), alanine (Ala), valine (Val), methionine (Met), isoleucine (Ile), leucine (Leu), tyrosine (Tyr), phenylalanine (Phe), histidine (His), tryptophan (Trp), lysine (Lys), and arginine (Arg).

4. The plant-based fermented product according to claim 1, wherein the fermented product is a fermented product of one and more plant-based materials selected from the group consisting of soybeans, peas, chickpeas, lentils, Fava beans, mung beans, rice, and purified proteins thereof.

5. The plant-based fermented product according to claim 1, wherein the fermented product is a fermented product of *Aspergillus oryzae* strain.

6. The plant-based fermented product according to claim 5, wherein the *Aspergillus oryzae* strain is *Aspergillus oryzae* SMF119 strain (deposit number KCTC12947BP).

7. The plant-based fermented product according to claim 1, wherein the fermented product is a fermented product of *Bacillus amyloliquefaciens* strain.

8. The plant-based fermented product according to claim 7, wherein the *Bacillus amyloliquefaciens* strain is *Bacillus amyloliquefaciens* SMB469 strain (deposit number KCTC12822BP).

9. A food composition comprising the plant-based fermented product according to any one of claims 1 to 8.

10. A flavoring material comprising the plant-based fermented product according to any one of claims 1 to 8.

11. The flavoring material according to claim 10, wherein the plant-based fermented product is comprised in an amount of 0.001 to 99.99% by weight based on the total weight of the flavoring material.

12. The flavoring material according to claim 10, wherein the flavoring material is added to a food product in an amount of 0.001 to 99.99% by weight based on the total weight of the food product.

13. The flavoring material according to claim 12, wherein the food product comprises at least 10% less sodium by weight based on the total weight of the food product compared to before the addition of the flavoring material.

14. A method for preparing the plant-based fermented product according to any one of claims 1 to 8, comprising: inoculating and culturing a plant-based material with *Aspergillus oryzae* strain; and inoculating and fermenting the cultured plant-based material with Bacillus amyloliquefaciens strain.

15. The method for preparing the plant-based fermented product according to claim 14, wherein the *Aspergillus oryzae* strain is *Aspergillus oryzae* SMF119 strain (deposit number KCTC12947BP).

16. The method for preparing the plant-based fermented product according to claim 14, wherein the step of inoculating and culturing the *Aspergillus oryzae* strain is performed at 20 to 40°C for 40 to 72 hours.

17. The method for preparing the plant-based fermented product according to claim 14, wherein the *Bacillus amyloliquefaciens* strain is *Bacillus amyloliquefaciens* SMB469 strain (deposit number KCTC12822BP).

18. The method for preparing the plant-based fermented product according to claim 14, wherein the step of inoculating and fermenting the *Bacillus amyloliquefaciens* strain is performed at 25 to 55°C for 5 to 200 days.

19. The method for preparing the plant-based fermented product according to claim 14, wherein the fermenting step comprises adding brine to the cultured plant-based material.

20. The method for preparing the plant-based fermented product according to claim 14, wherein the method further comprises a step of heat-treating the plant-based material before the culturing step.
